**Europäisches Patentamt**

**European Patent Office**

**Office européen des brevets**

(19)

(11) Publication number : **0 437 520 B1**

## EUROPEAN PATENT SPECIFICATION

(12)

(45) Date of publication of patent specification :
**15.04.92 Bulletin 92/16**

(51) Int. Cl.⁵ : **C11C 3/08, A61K 31/23**

(21) Application number : **89911780.8**

(22) Date of filing : **10.10.89**

(86) International application number :
**PCT/DK89/00234**

(87) International publication number :
**WO 90/04010 19.04.90 Gazette 90/09**

(54) **TRIGLYCERIDE AND NUTRITIONAL COMPOSITION COMPRISING SUCH TRIGLYCERIDE.**

(30) Priority : **10.10.88 DK 5652/88**

(43) Date of publication of application :
**24.07.91 Bulletin 91/30**

(45) Publication of the grant of the patent :
**15.04.92 Bulletin 92/16**

(84) Designated Contracting States :
**AT BE CH DE FR GB IT LI LU NL SE**

(56) References cited :
**WO-A-88/09325**
**US-A- 4 607 052**
**US-A- 4 701 468**
**US-A- 4 701 469**

(73) Proprietor : **NOVO NORDISK A/S**
**Novo Allé**
**DK-2880 Bagsvaerd (DK)**

(72) Inventor : **HANSEN, Tomas, Tage**
**Tonedraget 5**
**DK-3450 Aller d (DK)**
Inventor : **GODTFREDSEN, Sven, Erik**
**Smedegade 15B**
**DK-3500 V rl se (DK)**

(74) Representative : **Bach, Niels et al**
**c/o Novo Nordisk A/S Patent Dept. Novo Allé**
**DK-2880 Bagsvaerd (DK)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

# Description

The present invention relates to a triglyceride and a nutritional composition comprising such triglyceride.

Eicosapentaenoic acid, abbreviated EPA is an important fatty acid which is involved in vital processes in the body. For example, this fatty is a precursor in the biosynthesis of prostaglandins which control key events in the body such as blood pressure, or the tonus of smooth muscle tissues in the lungs. Furthermore EPA is involved in aggregation of blood platelets and is believed to exert a strong influence on artherosclerotic processes and other diseased states of the cardiovascular system.

Eicosapentaenoic acid or more precisely 5-8-11-14-17 all cis-eicosapentaenoic acid is biosynthesized in the body from $\alpha$-linolenic acid which by desaturation, elongation and desaturation provides EPA. In some individuals as e.g. elderly people or in connection with many diseased states this elongation and desaturation system is impared and supply of the essential fatty acid hom external sources becomes important. The EPA does occur in natural oils but it appears that absorption of EPA from natural sources is low. This is due, among other things, to specificities of the lipases involved in the metabolism of fats. It is thus well known that triglycerides containing long chain fatty acids only (e.g. EPA) are absorbed more slowly than the corresponding triglycerides containing medium chain length acids only ($C_8$-$C_{10}$) (Metabolism, Vol. 38, p. 507-513, 1989). Furthermore it is known that the human lipases, especially human pancreatic lipase, have a low activity towards polyunsaturated acids (e.g. EPA) (Lipids, Vol. 22, 711-714, 1987) and in particular towards $\omega$-3 fatty acids. This is the case also for the lipases as e.g. the lipoprotein lipase involved in cleavage of triglycerides applied as emulsions in parenteral nutrition. As mentioned above the only useful sources of EPA for enteral nutrition of the general public and e.g. diseased patients or elderly persons devoid of adequate desaturation and elongation activity have been mixtures of various EPA containing oils derived e.g. from plant materials. Since these raw materials contain mainly long chain fatty acids they are relatively poor as energy substrates and they provide the essential fatty acids as e.g. EPA in a poor bioavailable state when applied in enteral as well as in parenteral nutrition.

Thus, the purpose of the present invention is the provision of triglycerides which are devoid of the problems mentioned above regarding EPA containing triglycerides and which can thus be formulated as an enterally administrable composition, or as a nutrient exhibiting an improved bioavailability in regard to EPA, or which can be formulated as a parenterally administrable composition, which exhibits an improved bioavailability in regard to EPA besides being an efficient energy source.

A very important clinical aspect in regard to the bioavailability is the slower metabolism in comparison to emulsions containing MCT, which are known to cause metabolic acidosis, when infused intravenously.

The triglyceride according to the invention is 2-eicosapentaenoyl-1,3-didecanoyl glycerol. Surprisingly it has been found that this triglycerides exhibit a better bioavailability of EPA than the known EPA sources in nutrients, when formulated as an enterally administrable composition, and exhibits a better bioavailability than the known sources of EPA, when formulated as a parenterally administrable composition. Also, it has been found that the enterally administrable composition according to the invention can be better tolerated and digested and exhibits a better smell and a more optimal nutrition than the previoulsy known sources of EPA. Thus, the enterally administrable composition according to the invention exhibits a vastly reduced tendency to diarrhoeas. Moreover, the triglyceride of the invention turn out, surprisingly, to be a very efficient source of energy in enteral as well as in parenteral nutrition - the EPA moiety of the triglyceride being in an optimal position in the molecule in respect to its cleavage by lipoprotein lipase. Also, the triglyceride of the invention appears to allow preservation of the EPA in a hydrophobic form which can pass the intestinal mucosal layer and reach the enteroside in an optimal form for further esterification, lipoprotein transport and clearance.

Surprisingly, the triglyceride of the invention appear to exhibit physical properties which allow facile formulation of the compounds in liquid products as well as in powdered products exhibiting excellent wetability properties. In the liquid form the product of the invention possess excellent stabilities making sterilization of e.g. parenteral products containing the triglycerides of the invention reliable, easy and safe.

The triglyceride of the invention allows formulation of feeding products with a EPA meeting the requirements of the body making unnecessary use of large amounts of natural EPA containing oils which are sensitive to oxidation and polymerisation processes. Detrimental processes of polyunsaturated fats and oils leading to negative nutritional forms are thus commonly associated with gastric and intestinal problems due to oxidation and polymerisation products of the polyunsaturated fatty acid. Such oxidized forms of EPA can interfere with nitrogen uptake by interacting with sensitive amino acids in infant formula. These highly undesired effects are diminished or even avoided by applying triglycerides according to the invention.

For use in parenteral feeding the triglyceride according to the invention can be incorporated into lipid emulsions where the liquid phase amounts up to 30% of the emulsion and it can be processed using the usual techniques to provide chylomicronlike particles.

The triglyceride of the invention is advantageously applied in such emulsions due to its fast conversion by lipoprotein lipase and endothelial lipase and the consequential avoidance of the discomfort and side effects of lipolipaedemia. EPA applied in triglycerides of the invention is thus cleared quickly and efficiently thereby providing the essential fatty acid concomitantly with short chain acids useful as energy substrates.

The use of the triglyceride according to the invention in parenteral nutritional products is further particularly advantageous since the relatively high polarity of the triglyceride favour the stability of its emulsions which are subjected to severe heat treatments during their manufacturing. Use of such emulsion is particularly advantageous for nutrition of patients in which the desaturation and elongation system is insufficient to meet the needs.

A further advantage of the triglyceride of the invention is related to its solubility in other oils as e.g. vegetable oils. Such solutions can be used as such as a nutritional support. The triglyceride of the invention may also be formulated into creams and related products which allow the use of the compound for topical treatment of e.g. skin diseases associated with essential fatty acid deficiencies.

This invention is a selection invention in the sense that a general chemical formulation comprising the triglyceride according to the invention together with a very large number of other triglycerides belong to the prior art, vide FR 2515174, whereas the triglycerides according to the invention do not belong to the prior art. The triglyceride according to the invention is described and synthetized for the first time by the inventors, and also, the superior effect of the triglyceride according to the invention is demonstrated for the first time by the inventors.

An emulsion of the triglyceride 2-eicosapentaenoyl-1,3-dioctanoyl glycerol used for enteral application is described in US 4,607,052 and S. E. Boustani et al, Lipids, 22, 711-14 (1987). This triglyceride differs from and is in many respects inferior to the triglyceride according to the invention, and also, it is solely used for enteral purposes.

A preferred embodiment of the triglyceride according to the invention is characterized by the fact that it has a purity of at least 10%, preferably at least 30%, more preferably at least 50%, even more preferably at least 75%, and most preferably at least 90%. The higher the purity of the triglyceride, the higher the bioavailability of the of the triglyceride.

Also the invention comprises a nutritional composition, which comprises the triglyceride according to the invention. This composition can be either the triglyceride according to the invention without the other constituents which are necessary for a full nutritional composition, i.e. mainly vitamins, proteins and carbohydrates, or the triglyceride according to the invention together with these other constituents.

A preferred embodiment of the nutritional composition is parenterally administrable. This composition is an emulsion.

A preferred embodiment of the nutrional composition is enterally administrable. This composition is either an emulsion or an oil.

A preferred embodiment of the enteral composition according to the invention is in fluid form. The enteral composition can be an emulsion or a pure oil.

A preferred embodiment of the enteral composition according to the invention is in powder form, whereby the triglycerides are encapsulated or microencapsulated. One of the manners, in which the droplets of triglyceride can be encapsulated, is described in Danochemo Technical Information on microencapsulated Product, Malmparken 5, 2750 Ballerup, Denmark.

## Claims

1. The triglyceride 2-eicosapentaenoyl-1,3-didecanoyl glycerol.

2. Triglyceride according to Claim 1, which has a purity of at least 10%, preferably at least 30%, more preferably at least 50%, even more preferably at least 75%, and most preferably at least 90%.

3. Nutritional composition, which comprises the triglyceride according to Claim 1 or 2.

4. Nutritional composition according to Claim 3, which is parenterally administrable.

5. Nutritional composition according to Claim 3, which is enterally administrable.

6. Nutritional composition according to Claim 5, which is in fluid form.

7. Nutritional composition according to Claim 5, which is in powder form, whereby the triglycerides are encapsulated or microencapsulated.

## Patentansprüche

1. Das Triglycerid 2-Eicosapentaenoyl-1,3-didecanoylglycerin.

2. Triglycerid nach Anspruch 1, dadurch gekennzeichnet, daß es eine Reinheit von wenigstens 10 %, vorzugsweise wenigstens 30 %, bevorzugter wenigstens 50 %, noch bevorzugter wenigstens 75 % und am bevorzugtesten wenigstens 90 % besitzt.

3. Nahrungszusammensetzung, dadurch gekennzeichnet, daß sie das Triglycerid nach Anspruch 1 oder 2 umfaßt.

4. Nahrungszusammensetzung nach Anspruch 3, dadurch gekennzeichnet, daß sie parenteral verabreichbar ist.

5. Nahrungszusammensetzung nach Anspruch 3, dadurch gekennzeichnet, daß sie enteral verabreichbar ist.

6. Nahrungszusammensetzung nach Anspruch 5, dadurch gekennzeichnet, daß sie in flüssiger Form vorliegt.

7. Nahrungszusammensetzung nach Anspruch 5, dadurch gekennzeichnet, daß sie in Pulverform vorliegt, wobei die Triglyceride gekapselt oder mikrogekapselt sind.

## Revendications

1. Le triglycéride 2-eicosapentaénoyl-1,3-didécanoylglycérol.

2. Triglycéride selon la revendication 1 qui a une pureté d'au moins 10 %, de préférence d'au moins 30 %, mieux d'au moins 50 %, encore mieux d'au moins 75 %, et tout préférablement d'au moins 90 %.

3. Composition nutritionnelle qui comprend le triglycéride selon la revendication 1 ou 2.

4. Composition nutritionnelle selon la revendication 3 qui peut être administrée par voie parentérale.

5. Composition nutritionnelle selon la revendication 3 qui peut être administrée par voie entérale.

6. Composition nutritionnelle selon la revendication 5 qui est sous une forme fluide.

7. Composition nutritionnelle selon la revendication 5 qui est sous forme d'une poudre, les triglycérides étant encapsulés ou microencapsulés.